# EUROPEAN PATENT APPLICATION

(11) **EP 2 494 938 A1**
(43) Date of publication of application: **05.09.2012**
(21) Application number: 11156772.3
(22) Date of filing: 03.03.2011
(51) Int. Cl.: A61C 8/00

(54) **Dental implant assembly**

(71) Applicant: Astra Tech AB, 431 21 Mölndal (SE)
(72) Inventor: Holmström, Johan, 428 37, Kållered (SE); Andersin, Per, 435 41, Mölnlycke (SE); Magnusson, Daniel, 475 41, Hönö (SE)
(74) Representative: Somlo, Tommy

(57) **Abstract**

The present invention relates to a dental implant assembly, comprising a fixture for insertion into a jawbone which is provided with a first thread with a first thread start, and a dental component which is not axially symmetrical and which is provided at its apical end with a second thread with a second thread start having a rotational position which is known in relation to the asymmetrical shape of the dental component for engagement with the first thread. The fixture is provided with a distinguishable fixture index which has a known rotational position in relation to the first thread start. When the fixture has been inserted into the jawbone and the dental component has been fully installed therein, the rotational orientation of the fixture index in relation to the surrounding anatomy determines the rotational orientation of the asymmetrical shape of the dental component in relation to the surrounding anatomy. The invention also relates to a dental component and to a method of manufacturing a dental component.

## Description

### Field of the invention

The present invention relates to a dental implant assembly, comprising a fixture for insertion into a jawbone and a dental component, such as an abutment, an abutment replica, a driver, a healing cap or an impression pick-up element. The present invention also relates to a dental implant assembly, comprising a fixture for insertion into a jawbone and an installation device such as a driver. The present invention further relates to a dental fixture, a dental component and to a method for manufacturing a dental component to be installed in a fixture which is inserted in or is to be inserted in a jawbone of a patient.

### Background of the invention

Dental implant systems are widely used for replacing damaged or lost natural teeth. In such systems, a dental fixture is placed in the jawbone of a patient in order to replace the natural tooth root. An abutment structure comprising one or several parts may then be attached to the fixture in order to build up a core for the part of the prosthetic tooth protruding from the bone tissue, through the soft gingival tissue and into the mouth of the patient. On said abutment, the prosthesis or crown may finally be seated.

The final prosthesis should be sized and configured so as to naturally fit with the remaining teeth of the patient, both for functionality and aesthetics. To this end a dental technician may try out a proper prosthesis for the individual patient, using a model of the jaw of the patient, said model including the fixture. The dental technician may also digitally work out a proper prosthesis based on a digital model of the jaw of the patient either with a fixture already installed or prior to such installation. The dental technician may also modify a pre-fabricated abutment to match the contour of the soft gingival tissue.

There are various fixture configurations. For instance, a fixture may have a flat topped coronal head portion, which may be installed in any rotational position relative to the jawbone. Another type of fixture configuration is a fixture having a sloped coronal end portion, such as disclosed in US 6,655,961, in which the length of the fixture is greater on the lingual side than on the buccal side in order to match the contour of the jawbone.

Similarly, to the above described fixture/jawbone-interface, for a superstructure, such as an abutment, there may be an abutment/fixture-interface in which the abutment should only be positioned in one way relative to the fixture, e.g. an abutment having a sloped portion matching the sloped head portion of a fixture. This is one type of asymmetrical superstructure, in which the asymmetrical feature should be positioned in a desired rotational relationship to either or both of the fixture and the jawbone with surrounding teeth. Thus, with regard to these interfaces, it would be desirable to ensure that the dentist connects the superstructure with a correct rotational orientation relative to certain fixtures or jawbone features.

When making the abutment and the prosthetic tooth, the dental technician has thus taken the contours and locations of surrounding tissue and adjacent teeth into account. A system used at present time to manoeuvre the rotational position of asymmetrical superstructures is to provide a two-part abutment having a centrally positioned threaded screw, which mates with an internally threaded bore of the fixture, and a sleeve which is given the asymmetrical patient specific features at its coronal end which are needed to be a suitable basis for the prosthetic tooth. The sleeve may be rotated into the correct position in a seating of the internal bore of the fixture and be fixed therein by the screw. The seating in the fixture and the apical end of the sleeve of the abutment are axially symmetrical to ensure that a correct final position may be achieved. The correct positioning is this way depending on the dentist's ability to visually verify the result.

The dentist receives the abutment and prosthetic tooth either as one integral unit or as separate parts to be assembled in the oral cavity of the patient. Often, the dentist who receives the abutment and the prosthetic tooth should understand how the abutment should be rotationally oriented relative to the fixture in order to obtain the alignment as intended by the dental technician. Nevertheless, it may sometimes be difficult for the dentist to see which the correct orientation is and, of course, there may be a risk of the dentist simply overlooking or ignoring the correct rotational orientation of the abutment relative to the fixture.

It is an objective of the invention to mitigate the risk of a dentist connecting a dental component, such as an abutment, to a dental fixture with an incorrect rotational orientation than what was intended by the dental technician.

It is also an objective of the invention to mitigate the risk of a dentist positioning a dental component, such as an abutment, in a different rotational orientation in relation to the surrounding anatomy (such as jawbone, teeth etc.) than what was intended by the dental technician or manufacturer.

These and other objectives, which will become apparent in the following, are achieved by the implant assemblies, dental components, fixtures and methods as defined in the accompanied claims.

### Summary of the Invention

The present invention is based on the insight that by only having a single rotational orientation available for a fixture-mating arrangement (such a driver or a dental component in the form of an abutment, healing component etc.) when connected to a fixture, the dentist is prevented from inadvertently placing the fixture-mating arrangement in a different rotational orientation than what the dental technician or manufacturer had intended.

According to a first aspect of the invention, a dental implant assembly is provided. The dental implant assembly comprises a fixture for insertion into a jawbone which is provided with a first thread having a first thread start, and an axially asymmetrical dental component, such as an abutment, an abutment replica, a driver, a healing cap or an impression pick-up element, which is provided at its apical end with a second thread with a second thread start having a rotational position which is known in relation to the asymmetrical shape of the dental component for engagement with the first thread, wherein the fixture is provided with a distinguishable fixture index which has a known rotational position in relation to the first thread start, wherein, when the fixture has been inserted into the jawbone and the dental component has been fully installed therein, the rotational orientation of the fixture index in relation to the surrounding anatomy determines the rotational orientation of the asymmetrical shape of the dental component in relation to the surrounding anatomy.

Thus, said first thread is a component-connecting thread in contrast to bone-engaging threads which may also be provided on the fixture. Suitably, said first thread is provided in an internal bore or socket which is arranged at a coronal end of the fixture and which extends apically from said coronal end. An alternative would be to have said first thread on an exterior portion of the fixture, such as a summit portion intended to extend above the jawbone when the fixture has been finally installed.

The term rotational position is here and throughout the application intended to mean a position in relation to an axis of a component or a bore. Hence, rotational position may e.g. be a position somewhere at the surface of an internal bore or a position at an external surface of e.g. a dental component or a dental fixture.

The term rotational orientation is here and throughout the application intended to mean how two objects are rotationally oriented to each other. For example, consider a dental component having an engagement portion in the form of an external hexagon and being connected to a fixture having an internal bore with an internal hexagon as engaging portion. The dental component may be oriented in six different rotational orientations in relation to the fixture. If the engagement portion and engaging portion instead consists of double hexagonal fittings, the number of different rotational orientations are instead twelve. The two objects need not be a dental component and a dental fixture, it may also be e.g. a dental fixture or a dental component in relation to a jawbone or other surrounding anatomical structures, or a dental installation device in relation to a dental fixture.

The term index, as for example in fixture index mentioned above but also in installation device index and dental component index as will be mentioned below, is here and throughout the application intended to mean a characteristic that distinguishes a selected portion/feature of e.g. a fixture, an installation device or a dental component from the remaining portions of the fixture, installation device or dental component. The purpose of the different indexes described throughout the application is to give the user the possibility to identify the rotational positions of the thread starts of the different components mentioned above. Any one of the components mentioned above may be provided with more than one index. It is for example conceivable with several different colour markings identifying the index. It is for example also conceivable with several different indexes identifying the thread start of the thread. However, in this case the relationship between each one of the indexes and the thread start should all be different and the indexes should be distinguishable from each other. It is for example conceivable with different colour markings identifying different relationships with the thread start. For example, one index may be positioned at the same rotational position as the thread start, and another index may be positioned at a rotational position 180° from the thread start. Another index may be positioned at a rotational position 90° from the thread start in the clockwise direction and yet another index may be positioned at a rotational position 90° from the thread start in the counter clockwise direction. These other indexes may also be present for other reasons than identifying the rotational position of the thread start.

Furthermore, the characteristics defining an index may, but need not, be one single feature as long as the index is clearly distinguishable and informs the user of the rotational position of the thread start.

In the case of e.g. a contoured or customized dental component or fixture it may for example be the uppermost or lowermost part of the coronal end of the dental component or fixture that constitutes the index.

The term customized or patient-specific abutment is here and throughout the application intended to mean an abutment made specifically for a patient, taking into account the dental situation and anatomy in the patient's mouth, e.g. the shape of the patient's jawbone, the shape of the gingiva, and the shape and location of adjacent teeth.

With the term fully installed is here and throughout the application intended to mean that the dental component is provided at a predetermined axial position in relation to the fixture. There exist many different connections between fixtures and dental components, for example so called flat-to-flat connections where a flat lower surface of a dental component is intended to land on a flat upper surface of the fixture. There also exist conical connections in which a conical lower portion of a dental component is intended to land on a corresponding conical portion of an internal bore of the fixture. Another option is a conical connection in which the fixture has a male conical portion and the dental component has a female mating conical portion. Hence, the dental component is in these examples fully installed when it has landed on the mating surface of the fixture. In other words, when the dental component has reached its intended end position with respect to the fixture it is fully installed. Furthermore, the phrase "fully installed therein" is not limited to a portion of the dental component being inserted inside the fixture, e.g. in an internal bore or socket of the fixture, but also encompasses the alternative that the dental component is place around the fixture, e.g. the fixture having a male portion which fits into a female portion of the dental component.

When designing a dental component with an external thread to be engaged with the internal thread of a fixture, the travel length can be determined. The thread starts can be appropriately matched to each other and positioning in relation to the fixture index and the non-symmetrical shape of the dental component. Therefore, with regard to the fully installed state of the dental component in the fixture, the rotational orientation of said non―symmetrical shape relative to the fixture index can be derived in advance of connecting the dental component to the fixture.

The term axially asymmetrical or non-symmetrical dental component is here and throughout the application intended to mean a dental component which does not look the same if it is rotated by an angle about its central axis. In particular, the portion of the dental component that in use is intended to protrude outside the dental fixture to which the dental component is attached, is not axially symmetrical. Since it is the outside shape of the dental component that should be matched to the surrounding anatomy and/or the fixture, it should be understood that the asymmetry refers to the exterior or enveloping surface of the dental component, and not to the interior of the component. For instance, a circularly cylindrical component having an internal hexagonal recess would, in this application be considered an axially symmetrical component despite the internal recess.

The known relationship between the distinguishable fixture index and the first thread start means that it is possible for a user to know the rotational position of the first thread start while studying the fixture index. Hence, a dental technician knowing the rotational position of the first tread start (e.g. in an internal bore of the fixture) may design a dental component to be inserted into the bore with a matching thread and having a thread start at a desired rotational position, thereby enabling the dental component to be positioned in a known, desired and pre-defined rotational orientation relative to the fixture when fully installed therein. Furthermore, a dentist inserting the fixture into a patient's jawbone may, by use of the index, position the dental fixture in a desired position in relation to the jawbone. Hence, when the fixture is installed in a desired position and the rotational relationship between the fixture and the dental component, when fully installed therein, is known, the dentist will not have to determine in what rotational orientation the dental component should be attached to the fixture. Instead, the dentist will only have to connect the dental component to the fixture by using the respective screw threads, and the dental component will be correctly positioned.

Hence, it is with a dental assembly according to the first aspect of the present invention possible to already at the design stage of the dental component design it so that its asymmetrical shape is directed in the intended direction in relation to a dental fixture when fully installed therein and to insert the fixture in a patient's jawbone with a desired rotational orientation relative to the jawbone and other surrounding anatomy.

According to at least one exemplary embodiment, said rotational position of the first thread start is known in relation to the fixture. Since the rotational position of the first thread start is known in relation to the distinguishable fixture index, it is also known in relation to the fixture.

According to at least one exemplary embodiment, said fixture index is provided on an exterior of said fixture. An index provided at the exterior of the fixture is easily detectable for the user and may in certain embodiments be preferred.

According to at least one exemplary embodiment, said fixture index is located at the coronal end of the fixture. Providing the fixture index at the coronal end may allow the index to be visible also after the fixture has been inserted into a patient's jawbone, or at least during installation of the fixture. This may be useful in terms of simplifying for the dentist to position the index of the fixture in a desired pre-defined relationship to the patient's jawbone.

According to at least one exemplary embodiment, said fixture index is chosen from the group consisting of: an axially asymmetrical coronal fixture profile (or an area of such an axially asymmetrical coronal fixture profile), a recess, a protrusion, an indentation, a local surface modification and a colour marking.

With the term surface modification is here and throughout the application intended to mean e.g. a roughness of the surface different from the roughness of the surface for the remaining part of the fixture.

As understandable from the list of different possible fixture indexes, the index may either be a design feature of the fixture that also fulfils another purpose, such as an axially asymmetrical coronal fixture profile of e.g. a contoured or customized implant, or a special characteristic whose only purpose is to visualize for the user where the thread start is positioned. The fixture index may also be a local area of the axially asymmetrical coronal fixture profile, e.g. the coronal-most portion of a slope-topped fixture.

According to at least one exemplary embodiment, said fixture index is comprised in an internal bore extending apically from a coronal end of the fixture. It may for certain embodiments instead be preferred to provide the fixture index in the internal bore of the fixture. For instance, a colour marking might be visible through the gingival, which may be undesirable from an aesthetic perspective. Some implant manufactures prefer to have a smooth surface against the gingival in order to avoid possible irritation thereof. A fixture index on the interior of the fixture would not come in contact with the gingiva.

According to at least one exemplary embodiment, said fixture index is an asymmetrical irregularity in a surface of the internal bore. Providing the fixture index as an asymmetrical irregularity in a surface of the internal bore may be useful for certain applications. For example, when using an installation device for inserting the fixture, it is possible to design the installation device so that it, due to the asymmetrical irregularity of the internal bore, only fits in one pre-defined orientation in the fixture. The user, such as a dentist, may then be informed of the rotational orientation of the fixture in relation to the jawbone by studying the position of the installation device. This may be easier than to have to study the fixture index itself. Another application in which it may be useful with an asymmetrical irregularity of the internal bore is when the fixture is installed in the jawbone and an observation component, such as a scan abutment or other fixture locating object, is used to determine the orientation of the fixture. In order to determine the rotational orientation of the fixture, and in particular the rotational position of the first thread start, the observation component preferably only has one possible rotational orientation relative the fixture.

According to at least one exemplary embodiment, said dental component has a distinguishable dental component index on an exterior of said dental component, which has a known relationship with the rotational position of the second thread start.

The known relationship between the distinguishable dental component index and the second thread start is understood to mean that the second thread start has a rotational position which is known in relation to dental component index.

According to at least one exemplary embodiment, said dental component index is chosen from the group consisting of: a dental component shoulder profile, a fixture adjoining surface profile, an asymmetrical feature, a recess, a protrusion, an indentation, a local surface modification and a colour marking. Hence, the dental component index may be a characteristic in the dental component also fulfilling another purpose or it may be a characteristic whose only purpose is to inform a user of the rotational position of the second thread start. It is also conceivable with a combination of distinguishable dental component indexes. For example, the dental component is not axially symmetrical and has e.g. an axially asymmetrical should profile. However, it may in certain embodiments be beneficial in terms of simplifying the work for a dentist or a dental technician if the dental component is also provided with e.g. a colour marking.

According to at least one exemplary embodiment, said dental implant assembly comprises at least two dental components, wherein the rotational position of the second thread start is different between the at least two dental components, in relation to their respective asymmetrical shapes, thereby enabling said asymmetrical shapes to be positioned in a respective rotational orientation relative to the fixture when the dental components are fully installed therein.

It may for certain applications be beneficial if the dentist has the possibility to choose between different dental components. If the different dental components in a set has a different rotational position of their respective thread starts in relation to the asymmetric shape of the dental component, the dentist may choose the dental component giving the best fit with the patient's anatomy depending on the rotational position of the fixture in relation to the anatomy.

According to one exemplary embodiment, said dental implant assembly comprises at least two dental components, wherein the rotational position of the second thread start is the same for the at least two dental components, in relation to their respective non-symmetrical shapes, and wherein the non-symmetrical shape of the at least two dental components is different. Hence, it is with a set according to this embodiment possible for the dentist to choose between dental components having different non-symmetrical shapes in order to choose the non-symmetrical shape giving the best fit with the patient's jawbone. However, according to this embodiment, each one of the at least two embodiments will be positioned similarly relative to the fixture. For instance, the dental components may be in the form of abutments having sloping shoulders. The shoulders may be inclined in the same direction with respect to the thread starts, but one of the abutments may e.g. have a steeper sloping shoulder than the other.

According to at least one exemplary embodiment, said dental component is a one-piece component.

The term one-piece component is here and throughout the application intended to mean a component providing both fastening to the fixture and providing the function of the component in one single piece.

By providing the dental component as a one-piece component having the features described for the first aspect of the present invention, the problems related to two-piece components described under the heading Background of the invention are alleviated.

According to at least one exemplary embodiment, said fixture is provided with external threads for engagement with a bore in said jawbone, and wherein said external threads have a thread start having a rotational position which is known in relation to the fixture index.

By designing the external threads of the dental fixture so that the thread start is known in relation to the dental fixture, it may be more convenient in order for the dentist to position the fixture in a desired rotational orientation in relation to the jawbone.

The external thread may be a multiple thread. In that case the position of the thread starts are known in relation to the fixture index.

According to a second aspect of the present invention, a dental implant assembly is provided. The dental implant assembly comprises a fixture for insertion into a jawbone, which is provided with an engaging portion having a first thread with a first thread start having a rotational position which is known in relation to the engaging portion, and an installation device, such as a driver, which is provided at an apical end with a first engagement portion which is adapted to mesh in a pre-defined rotational orientation with said engaging portion of the fixture during installation thereof, the installation device further comprising a distinguishable installation device index having a known rotational position to the first engagement portion.

Thus, said first thread is a component-connecting thread in contrast to bone-engaging threads which may also be provided on the fixture. Suitably, said first thread is provided in an internal bore or socket which is arranged at a coronal end of the fixture and which extends apically from said coronal end. An alternative would be to have said first thread on an exterior portion of the fixture, such as a summit portion intended to extend above the jawbone when the fixture has been finally installed.

By providing an implant assembly according to the second aspect of the invention, it is possible to position the engaging portion of the fixture in a known rotational orientation relative to the jawbone when fully engaged therein. A dental component described in accordance with the first aspect of the present invention may thereafter be attached to the fixture, and having the features of the dental components described in accordance with the first aspect of the present invention, become positioned in the desired rotational orientation in the fixture in the patient's jawbone.

By the term fully engaged is here and throughout the application intended to mean the desired final position of the dental fixture in relation to the jawbone. Hence, the position in which the dental fixture is left in the jawbone in order to become osseointegrated therein.

Since the installation device is adapted to be connected to the fixture in only one rotational orientation, the fixture need not be provided with a fixture index. Instead, the index at the installation device will inform the user, e.g. a dentist, the rotational orientation of the first thread start as the fixture is inserted into the bone. Once the installation device is removed from the fixture it may not be possible to determine the rotational orientation of the first thread start in relation to the patient's jawbone. However, since the dental component according to the first aspect of the present invention is designed to be correctly positioned in the fixture, the dentist will only have to engage the threads of the dental component with the threads of the fixture, and the dental component will, when fully installed in the fixture, become correctly oriented.

It is of course conceivable with exemplary embodiments in which the fixture is provided with a fixture index in combination to the installation device index.

The installation device may in certain exemplary embodiments be connected or engaged with the fixture by means of the respective threads. In other exemplary embodiments, the installation device may be engaged with the fixture in any other suitable manner known in the art as long as it only can be engaged in one known/pre-defined rotational orientation, e.g. through an asymmetrical outline or distribution of recess/protrusions.

According to at least one exemplary embodiment, said rotational position of the first thread start is known in relation to the fixture.

According to at least one exemplary embodiment, said installation device index is chosen from the group consisting of: a fixture adjoining surface profile, a non-symmetrical feature, a recess, a protrusion, an indentation, a local surface modification and a colour marking.

The main purpose of the installation device index is to visualize for the user where the thread start of the first thread is positioned. However, as mentioned above for the first aspect of the present invention, the index may also be a characteristic fulfilling another purpose of the installation device.

According to at least one exemplary embodiment, said dental implant assembly further comprises an axially asymmetrical dental component, such as an abutment, an abutment replica, a healing cap or an impression pick-up element, to be installed in said fixture, said dental component being provided at an apical end with a second engagement portion having a second thread with a second thread start having a rotational position for engagement with the first thread, wherein the rotational position of said second thread start is known in relation to the asymmetrical shape of the dental component, thereby enabling the dental component to be positioned in a known rotational orientation relative to the fixture when installed therein.

When the fixture is installed in the patient's jawbone a dental component may be attached thereto. With a dental component according to this exemplary embodiment, a dental technician may design the dental component so that the asymmetrical shape of it becomes correctly positioned in the fixture when the dentist installs it in the fixture by engaging the first and second threads with each other to the fully installed position.

According to at least one exemplary embodiment, said dental implant assembly comprises at least two dental components, wherein the rotational position of the second thread start is different between the at least two dental components, in relation to their respective asymmetrical shapes, thereby enabling said asymmetrical shapes to be positioned in a respective rotational orientation relative to the fixture when the dental components are fully installed therein.

Similar to the exemplary embodiment described for a set of at least two different dental components in relation to the first aspect of the present invention, it may be beneficial to provide such a set also for the second aspect of the present invention.

According to at least one exemplary embodiment, the first engagement portion has a third thread having a third thread start having a rotational position for engagement with the first thread.

The installation device is, according to the second aspect of the present invention, only able to mesh with the fixture in one pre-defined rotational orientation. One possible manner in achieving this limited possibility of meshing the installation device with the fixture is to provide the engagement portion of the installation device with threads that are adapted to engage the first threads of the fixture. When the third thread of the installation device is fully engaged with the first thread of the fixture, i.e. has reached a stop position in relation to the fixture, it can only be positioned in one rotational orientation. It may therefore in certain exemplary embodiments be beneficial to utilize the first threads of the fixture for engaging the installation device.

According to at least one exemplary embodiment, the fixture is provided with a distinguishable fixture index which has a known rotational position in relation to the engaging portion.

Even though the installation device is provided with an installation device index which visualizes for the user, e.g. a dentist, the position of the first thread start when the fixture is installed in the jawbone, it may for certain exemplary embodiments be beneficial if the fixture is provided with a fixture index visualizing the rotational position of the engaging part. Since the rotational position of the first thread start in relation to the engaging part is known according to the second aspect of the present invention, the rotational position of the first thread start becomes known in relation to the fixture index. This may for example be the situation when it is desirable to determine the position of the first thread start after the installation device has been removed from the fixture, for example when a scan is to be taken of the dental situation of the patient's jawbone after the fixture has been inserted.

According to at least one exemplary embodiment, said fixture index is comprised in an exterior of said fixture. According to at least one exemplary embodiment, said fixture index is located at the coronal end of the fixture. According to at least one exemplary embodiment, said fixture index is chosen from the group consisting of: an axially asymmetrical coronal fixture profile (or a local area of such an axially asymmetrical coronal fixture profile), a recess, a protrusion, an indentation, a local surface modification and a colour marking. According to at least one exemplary embodiment, said dental component is a one-piece component. According to at least one exemplary embodiment, said fixture is provided with external threads for engagement with a bore in said jawbone, and wherein said external threads have a thread start having a rotational position which is known in relation to the engaging portion. The advantages of and different alternatives within the exemplary embodiments described above with respect to the second aspect of the invention are similar to the advantages and different alternatives as described above for the corresponding exemplary embodiments of the first aspect of the invention.

According to a third aspect of the present invention, an axially asymmetrical dental component, such as an abutment, an abutment replica, a healing cap or an impression pick-up element, to be installed in a fixture which is installed in or is to be installed in a jawbone of a patient is provided, said fixture being provided with a first thread having a first thread start, wherein the first thread start has a known rotational position in relation to a distinguishable fixture index,
the dental component being provided at an apical end with a second thread with a second thread start having a rotational position which is known in relation to the asymmetrical shape of the dental component, for engagement with the first thread, the position of the second thread start being adapted to the position of the first thread start such that prior to installation of the dental component in the fixture the rotational orientation between the asymmetrical shape of the dental component and the fixture index after final installation is known.

Thus, said first thread is a component-connecting thread in contrast to bone-engaging threads which may also be provided on the fixture. Suitably, said first thread is provided in an internal bore or socket which is arranged at a coronal end of the fixture and which extends apically from said coronal end. An alternative would be to have said first thread on an exterior portion of the fixture, such as a summit portion intended to extend above the jawbone when the fixture has been finally installed.

The known relationship between the distinguishable fixture index and the first thread start means that it is possible for a user to know the rotational position of the first thread start while studying the fixture index. Hence, a dental technician knowing the rotational position of the first tread start (in the internal bore or on an exterior summit portion) may design a dental component with a matching thread and having a tread start at a desired rotational position, thereby enabling the dental component to be positioned in a known, desired and pre-defined rotational orientation relative to the fixture when fully installed therein. Hence, a dentist will only have to connect the dental component to the fixture by using the respective screw threads, and the dental component will become positioned in the pre-defined orientation.

The possibility to design a dental component knowing that it will become positioned in a pre-defined rotational orientation in the fixture is useful e.g. when designing customized abutments.

When a dental surgery for a patient is planned, one may in advance determine the desired rotational orientation of the asymmetrical dental component with regard to the anatomy which will surround the dental component. Since the rotational orientation of the asymmetrical shape of the dental component (when fully installed in the fixture) is known in relation to the fixture index, one may in advance determine the appropriate rotational orientation of the fixture index with respect to the anatomy. Thus, as long as the dentist inserts the fixture with the instructed rotational orientation in relation to the surrounding anatomy, the dental component will, when fully installed, obtain the planned rotational orientation.

According to at least one exemplary embodiment, said dental component is a one-piece component. The advantages of and different alternatives within this exemplary embodiment is similar to the advantages and different alternatives as described above for the corresponding exemplary embodiments of the first and second aspects of the invention.

According to a fourth aspect of the present invention, there is provided a method for manufacturing an axially asymmetrical dental component, such as an abutment, an abutment replica, a healing cap or an impression pick-up element, which is to be installed in a fixture which is installed in or is to be installed in a jawbone of a patient, said fixture being provided with a first thread with a first thread start, the method comprising the steps of:
- providing the dental component at an apical end with a second thread with a second thread start having a rotational position which is known in relation to the asymmetrical shape of the dental component, for engagement with the first thread, and
- adapting the second thread start to the position of the first thread start such that a desired rotational orientation between the asymmetrical shape of the dental component and the fixture after final installation is achieved.

Thus, said first thread is a component-connecting thread in contrast to bone-engaging threads which may also be provided on the fixture. Suitably, said first thread is provided in an internal bore or socket which is arranged at a coronal end of the fixture and which extends apically from said coronal end. An alternative would be to have said first thread on an exterior portion of the fixture, such as a summit portion intended to extend above the jawbone when the fixture has been finally installed.

With the method for manufacturing an axially asymmetrical dental component according to the fourth aspect of the present invention, a dental technician may design a dental component that will become positioned in the desired rotational orientation. This is because the dental technician knows the desired or pre-determined rotational orientation that the fixture will have once installed in the patient's jawbone, and the dental technician may thereafter adapt the thread start of the dental component so that the non-symmetrical shape of the dental component becomes oriented in the desired rotational position once it is fully installed in the fixture. Hence, the designing and manufacturing of the dental component may be performed before the dental fixture is inserted into a patient's jawbone.

The fixture does not need to be provided with a fixture index according to the fourth aspect of the present invention. It is for example conceivable that the orientational relationship between the fixture and the first thread start is known by the use of an installation device as described in accordance with the second aspect of the present invention.

The method according to the fourth aspect of the invention also encompasses the situation when a fixture is already inserted into the jawbone of a patient. In such case, the dental component may be designed and manufactured based on, e.g. a digital of physical impression or a model of the jawbone and surrounding anatomy. The dental component may then be processed from a blank by means of an computerized process, or alternatively a technician may manually re-shape a stock component based on the model of the jawbone which suitably also presents a fixture replica.

Furthermore, in the method according to the fourth aspect of the invention, the fixture installed in the jawbone does not have to be provided with a distinguishable fixture index. Instead, a separate connectible observation component having a distinguishable observation component index may be used for determining how to adapt the second thread start of the dental component to the asymmetrical shape of the dental component. This is reflected in at least one example embodiment.

Thus, according to at least one example embodiment, the method according to the fourth aspect comprises
- providing an observation component comprising a distinguishable observation component index and a thread with a thread start, wherein the rotational position of the thread start of the observation component is known in relation to the distinguishable observation component index, wherein the thread of the observation component is adapted to engage with said first thread of the fixture for installing the observation component into or onto the fixture,
- observing, when the observation component has been fully installed in/on the fixture installed in the jawbone, the rotational orientation of the observation component index in relation to the surrounding anatomy,
wherein said step of adapting the second thread start to the position of the first thread start is based on the observed rotational orientation of the observation component index in relation to the surrounding anatomy.

Thus, when the rotational orientation of the observation component relative to the surrounding anatomy has been observed, it is also possible to deduce the rotational orientation of the thread start of the observation component. Because the length of the thread of the observation component is also known, the rotational orientation of the first thread start of the fixture in relation to the surrounding anatomy is also deducible. However, the dental technician does not necessarily have to know the rotational orientation of the first thread start, but could simply provide a dental component having a threaded portion corresponding to the threaded portion of the observation component. Because the rotational orientation of the thread start of the observation component in the fully installed state is deducible, by having a corresponding threaded portion on the dental component the dental technician will know that the second thread start of the dental component will in the fully installed state of the dental component, have the same rotational orientation. Based on this, the dental technician can then design the asymmetrical shape of the patient-specific dental component so that the rotational orientation of said asymmetrical shape will be correctly oriented when the dental component has been fully installed.

According to at least one exemplary embodiment, the fixture is provided with a distinguishable fixture index having a known relationship to the first thread start, the step of manufacturing the dental component being preceded by the steps of:
- observing a rotational orientation of said fixture based on said fixture index,
- determining a desired rotational orientation of said asymmetrical shape of the dental component in relation to the fixture when fully installed therein,
- selecting the rotational position of the second thread start of the dental component corresponding to the desired rotational orientation of said dental component in relation to the fixture.

The method according to this exemplary embodiment may be performed either before, during or after the fixture is installed in the patient's jawbone.

According to at least one exemplary embodiment, the step of observing a rotational orientation of said fixture is performed prior to installing the fixture in the jawbone and includes:
- observing a jawbone of a patient, and
- determining a desired rotational orientation of the fixture to be installed in said jawbone when it is fully installed therein.

According to at least one exemplary embodiment, the step of observing a jawbone of a patient includes scanning the jawbone using digital scanning equipment.

The determination of the rotational orientation of said fixture index (and suitably also the inclination of the fixture and the height/depth and/or buccolingual and/or mesiodistal positions of the fixture with respect to the jawbone) may be achieved by means of an acquired intra-oral impression of the implantation site. Therefore, in accordance with at least one example embodiment, the method comprises: receiving an intra oral impression of an implantation site at the maxilla or mandible in which a dental fixture having said fixture index has been inserted, wherein an inclination of the fixture relative to the jawbone and the rotational orientation of said fixture index relative to the jawbone are derivable from said impression.

Said impression may either be a physical impression, i.e. impression material is applied to the implantation site and the surrounding teeth, and then the impression material is let to harden so as to mimic the shape of the teeth. However, said impression may, alternatively, be a digital (optical) impression provided by means of an electronic scanning apparatus. In the letter case, a set of data would provide a representation of the implantation site and adjacent teeth. Said set of data may be viewable as an image on a user interface, such as a screen associated with a computer. Examples of electronic scanning apparatuses that may be used are various types of 3D scanners. For instance, a contact 3D scanner may probe the implantation site through physical touch. A CCM (Coordinate Measuring Machine) is an example of a contact 3D scanner. Alternatively, a non-contact active scanner may be used, such as a laser scanner or a light scanner. A further alternative is non-contact passive scanning, such as a stereoscopic system which may include two video cameras slightly apart. Yet another alternative is CT-scanning (Computed Tomography).

An alternative to acquiring an impression of an implantation site for determining the inclination of the inserted fixture and the rotational orientation of said fixture index, is to determine such inclination and rotational orientation before inserting the fixture into the jawbone. For instance, the direction of the bore hole in the jawbone into which the fixture is to be inserted may be planned in advance, and suitably, also the depth of the bore hole. It is also possible to plan in advance in which orientation the fixture index of the fixture should be arranged when the fixture is implanted. For instance, if a driver can only mate with the fixture in one rotational orientation, the rotational orientation of the fixture index may be derived via, for instance, a distinctive marking on the driver. Thus, when the fixture is driven into the bore hole, which has been prepared with the planned inclination, the dentist can, when installing the fixture in the bore hole, decide by looking at the distinctive marking on the driver when the fixture index is in the planned rotational position. Because both the inclination of the fixture and the rotational orientation of the fixture index can be planned in advance, it is effectively possible to provide the dentist with the fixture and the fixture-mating dental component (e.g. abutment, final restoration, etc) in one go.

Irrespective of whether the inclination of the fixture and rotational orientation of said fixture index is pre-planned before installation of the fixture, or based on a received impression which is either a physical impression or a digital impression, the inducible information may be used for providing a model of the implantation site.

Thus, according to at least one example embodiment of the invention, the method comprises providing a model of the implantation site with adjacent teeth in the jaw based on a determined inclination of the fixture and rotational orientation of said fixture index, the model comprising a fixture replica having the corresponding inclination and orientation of fixture index relative to the model as the fixture has or will have relative to the jawbone. The model may be a physical model, such as made from plastic, plaster, etc. The fixture replica could be a separate part which is installed into the rest of the model. Alternatively, the fixture replica could be made from the same material as the rest of the model, e.g. if the model is formed by sintering or machining based on a data file. As another option, rather than having a physical model, the model may be digital. A digital model may be obtained by first scanning the oral cavity in order to capture a digital impression, and then inputting the data representing the digital impression into a CAD program. The fixture replica in the digital model will thus be a virtual fixture, or at least a part of a virtual fixture comprising said fixture index.

A dental component or a blank may be installed in a traditional physical model including a fixture replica. The dental technician may then shape said dental component into the axially asymmetrical shape that he/she considers appropriate in view of the model.

Rather than manually shaping the dental component, an alternative would be to determine the shape with e.g. a CAD program. Thus, according to at least one example embodiment, the formed model is scanned, and the scanned model is transformed into a three dimensional digital image, wherein the determination of the shape of the dental component is based on the three dimensional image. The three dimensional image does not only allow the dental component such as an abutment to be designed from a computer, but also the final tooth shape may be designed in such manner. Thus, when the tooth shape has been decided, the abutment may be designed, and then the abutment may be produced in accordance with the design. As an alternative to scanning the model, it would be conceivable to scan the actual physical impression and from that create a digital model and determine the shape of the abutment.

As an alternative to scanning a physical model, it would be conceivable to create a virtual model, e.g. a three-dimensional CAD model based on data input representing a digital impression.

According to at least one example embodiment, the manufacturing method comprises
- inputting data of the CAD (computer aided design) model into a CAM (computer aided manufacturing) software, and
- controlling a dental component forming process with said CAM software, wherein the forming processes is selected from the group consisting of:
   a) additive manufacturing, such as fused deposition or laser sintering, and
   b) subtractive manufacturing, such as machining or milling a blank.

A dental component may be precision-machined from a solid blank.

The blank may have a pre-fabricated apical threaded portion, or alternatively, the threaded portion may be formed when processing the blank. A dental component in the form of an abutment may be precision-machined from a solid blank of e.g. medical grade titanium alloy or zirconia.

According to a fifth aspect of the present invention, there is provided a method for manufacturing an axially asymmetrical dental component, such as an abutment, an abutment replica, a healing cap or an impression pick-up element, which is to be installed in a fixture which is installed in a jawbone of a patient, said fixture being provided with an engaging portion having a first thread with a first thread start having a rotational position which is known in relation to the engaging portion, the method comprising the steps of:
- observing the rotational position of said first thread start by using an observation component which is installed in said fixture when said fixture has been installed in the jawbone of the patient, said observation component being provided at an apical end with an engagement portion which is adapted to mesh in a pre-defined rotational orientation with said engaging portion of the fixture, the observation component further comprising a distinguishable observation component index having a known rotational position in relation to the engagement portion,
- determining a desired rotational orientation of said asymmetrical shape of the dental component in relation to the fixture when fully installed therein,
- selecting the rotational position of the second thread start of the dental component corresponding to the desired rotational orientation of said asymmetrical shape of the dental component in relation to the fixture,
- providing the dental component at an apical end with a second thread with a second thread start having the selected rotational position in relation to the dental component.

Thus, said first thread is a component-connecting thread in contrast to bone-engaging threads which may also be provided on the fixture. Suitably, said engaging portion and first thread is provided in an internal bore or socket which is arranged at a coronal end of the fixture and which extends apically from said coronal end. An alternative would be to have said engaging portion and first thread on an exterior portion of the fixture, such as a summit portion intended to extend above the jawbone when the fixture has been finally installed.

According to at least one exemplary embodiment, the observation component is a scan abutment or other fixture locating object. Another alternative is to configure an installation device, such as a driver, to function as an observation component. A further alternative is a temporary abutment. According to at least one exemplary embodiment, the step of observing the rotational orientation of the first thread start includes scanning the observation component using digital scanning equipment.

According to at least one exemplary embodiment, the method further comprises the step of adapting the dental component to the jawbone of the patient.

Any features and example embodiments presented in relation to the fourth aspect of the invention may be incorporated in the method according to the fifth aspect of the invention, if compatible therewith.

According to a sixth aspect of the present invention, a dental fixture for insertion into a jawbone is provided. The dental fixture is provided with a first thread with a first thread start having a rotational position, wherein the fixture is provided with a distinguishable fixture index which has a known rotational position in relation to the first thread start.

Said first thread is a component-connecting thread in contrast to bone-engaging threads which may also be provided on the fixture. Suitably, said first thread is provided in an internal bore or socket which is arranged at a coronal end of the fixture and which extends apically from said coronal end. An alternative would be to have said first thread on an exterior portion of the fixture, such as a summit portion intended to extend above the jawbone when the fixture has been finally installed.

The known relationship between the distinguishable fixture index and the rotational position of the first thread start means that it is possible for a user, e.g. a dental technician, to know the rotational position of the first thread start while studying the fixture index.

For a dental technician knowing the rotational position of the first tread start in the internal bore or on a summit portion, it is possible to design a dental component with a matching thread and having a tread start at a desired rotational position, thereby enabling the dental component to be positioned in a known or desired rotational orientation relative to the fixture when fully installed therein.

According to at least one exemplary embodiment, said fixture index is comprised in an exterior of said fixture. According to at least one exemplary embodiment, said fixture index is located at the coronal end of the fixture.

According to at least one exemplary embodiment, said fixture index is chosen from the group consisting of: an axially asymmetrical coronal fixture profile (or a local area of such a profile), a recess, a protrusion, an indentation, a local surface modification and a colour marking. According to at least one exemplary embodiment said fixture index is comprised in the internal bore. According to at least one exemplary embodiment, said fixture index is a non-symmetrical irregularity in a surface of the internal bore. According to at least one exemplary embodiment, said fixture is provided with external threads for engagement with a bore in said jawbone, and wherein said external threads have a thread start having a rotational position which is known in relation to the fixture index.

According to at least one exemplary embodiment, the fixtures mentioned above for any one of the second, fourth, fifth and sixth aspect of the present invention comprises the features of the fixture mentioned for the first aspect of the present invention. The advantages of and different alternatives within the exemplary embodiments described above are similar to the advantages and different alternatives as described above for e.g. the corresponding exemplary embodiments of the first aspect of the invention.

The first, second and third threads mentioned for the different aspects of the present invention are, according to one exemplary embodiment, single threads. The purpose of the first, second and third threads are to provide a desired or known orientational relationship between different dental components and the fixture.

In this application it should be understood that a dental implant or dental implant assembly may comprise a dental fixture and a superstructure, such as an abutment.

A dental fixture is for use as the anchoring member of a dental prosthesis. To this end, the dental fixture is insertable into a pre-prepared bore hole in the bone tissue of a jawbone (maxilla or mandible) at a site where the dental prosthesis is required. The dental fixture is normally rotated into the bore hole.

For screw-type dental fixtures the bore hole may be provided with internal threads in advance or may be left un-tapped with the dental fixture provided with a self-tapping capacity, e.g. by the provision of one or more axially-extending cutting recesses, edges or notches, etc in the fixture thread. For instance, an apical end portion of the fixture may be provided with 2-4 cutting recesses, such as 3 cutting recesses. Other number of cutting recesses are readily conceivable.

A superstructure for connecting a prosthetic part to the fixture may comprise an abutment, spacer or other transmucosal component which engages to the dental fixture to bridge the gingiva overlying the maxilla or mandible. The prosthetic part, e.g. a crown, bridge or denture may be secured to the abutment. There are various other forms that the superstructure can take. For instance, the prosthetic part may be secured directly to the dental fixture. A dental implant may thus comprise an abutment connected to the dental fixture, or the dental fixture without an abutment.

The term "coronal" is here and throughout this application used to indicate a direction towards a head end or trailing end of the dental implant. For instance, in a situation where an abutment is connected to a dental fixture, the coronal direction of the abutment would be a direction towards the part of the abutment being directed away from the fixture. Conversely, the term "apical" indicates a direction towards an insertion or leading end of the component. Thus, apical and coronal are opposite directions. Furthermore, the terms "axial", "axial direction" or "axially" are used throughout this application to indicate a direction taken from the coronal end to the apical end, or vice versa. The terms "radial", "radial direction" or "radially" indicate a direction perpendicular to the axial direction.

A blind bore or socket may extend apically into the fixture body from the coronal end to an end surface in-between the apical and coronal ends of the fixture body for a superstructure to be secured to the fixture. The socket may comprise an internally-threaded section for screw connection of the superstructure to the fixture. A rotational lock for the superstructure may be provided in the socket, such as an internal polygonal side wall, e.g. hexagonal, or alternatively one or more protrusions from or indentation in the wall of the socket. A section of the socket, such as the coronal section, may be tapered towards the apical end. The tapered section is suitably arranged coronally of the internally-threaded section.

The fixture may be used in a one stage procedure or a two stage procedure. In a one stage procedure a healing or temporary abutment is connected to the fixture to form the gingival tissue, and after a healing period the healing or temporary abutment is replaced by a permanent abutment. For a two stage procedure the fixture is provided with a cover screw and the gingival tissue is sutured over the fixture and cover screw, and after a healing period the tissue is opened up and an abutment is connected to the fixture after removal of the cover screw.

The fixture may have a conically tapering end portion which tapers towards the coronal end. The axial extent of this coronal end portion is small compared to the total length of the fixture, as an example no more than 4 % of the total length, such as in the range of 1.5% -3.7%. The coronal end portion may suitably be provided without a threaded surface, e.g. having a smooth or a roughened (such as blasted) surface.

The fixture may have a substantially flat coronal end surface which is perpendicular to the longitudinal axis of the fixture. Alternatively, the coronal end surface may have a sloped contour relative to the longitudinal axis of the fixture, e.g. such that when positioned within the jawbone the length of the fixture is larger on a lingual side and shorter on a buccal side of the fixture. Another alternative is a saddle-shaped or wave-like coronal end surface.

The length of the dental fixture may be in the range of 5-19 mm, depending on the clinical situation. The outer diameter of the dental fixture may suitably be in the range of 2-6 mm, such as 3-5 mm.

The fixture may be substantially cylindrical or slightly tapering from the coronal end towards the apical end. If the fixture has a slight tapering, the core of the fixture and the outer periphery defined by e.g. thread tops may have the same or different angle of taper. Furthermore, the core of the fixture may be cylindrical while the thread tops describe a conicity or, conversely, the core of the fixture may be tapered while the thread tops describe a generally cylindrical geometry. Alternatively, the fixture may comprise a combination of one or more cylindrical and/or one or more tapering portions. Thus, one or more portions of the fixture may have e.g. thread tops lying in a common imaginary cylindrical surface, which cylindrical surface is parallel with the longitudinal axis of the fixture. Alternatively or additionally, one or more portions of the fixture may have thread tops lying in an imaginary conical surface which in the apical direction is tapering towards the longitudinal axis.

The externally threaded fixture may comprise one or more thread spirals.

The term "pitch" is used to indicate the axial distance between adjacent tops of a threading. The term "lead" is used to indicate the distance advanced parallel to the longitudinal axis when the fixture is turned one revolution, i.e. it corresponds to the pitch multiplied with the number of thread spirals. For a single thread spiral having a constant pitch, the lead is equal to the pitch; for a double thread spiral, the lead is twice the pitch.

The term "microthread" is used to indicate a thread having a height which is no greater than 0.2 mm. According to at least one example embodiment, the fixture is provided with microthreads having a height in the range of 0.02-0.2 mm, such as 0.05-.015 mm, for instance 0.1 mm. The term "macrothread" is used to indicate a thread having a height which is greater than 0.2 mm. According to at least one example embodiment, the fixture is provided with macrothreads having a height in the range of 0.25-0.35 mm, such as 0.3 mm.

Suitably, microthreads may be located coronally of macrothreads. For instance, microthreads may be arranged to engage dense cortical bone and macrothreads may be arranged to engage porous spongious/cancellous bone. The lead of a microthread suitably corresponds to the lead of a macrothread. The macrothread pitch may, as an example, be 2-4 times, such as 3 times, the pitch of the microthreads. The pitch (top-to-top spacing) at a fixture portion provided with microthreads may be around 0.20-0.24 mm. The pitch (top-to-top spacing) at a fixture portion provided with macrothreads may be around 0.60-0.72 mm.

Microthreads can be regarded as defined, oriented roughness. A non-oriented roughness having smaller dimensions, for instance obtained by blasting, etching, etc., may be superimposed on microthreads as well as on macrothreads.

A thread profile comprises two flanks, a top radius R, at the apex formed between the intersection of said two flanks, a bottom radius r formed between two adjacent threads, said flanks forming an angle v with a plane which is perpendicular to a cross section of said thread and perpendicular to a plane which is a tangent to the surface of the fixture body, said profile further having a height D. Suitably for 10° ≤ v < 35 °, R is greater than 0.4 x D and, for 35° ≤ v < 55 °, R is greater than 0.2 x D.

### Brief description of the drawings

Fig. 1 illustrates a dental implant assembly according to at least one example embodiment.
Fig. 2 is a cross-sectional view of the implant assembly in Fig. 1.
Figs. 3 and 4 illustrate a dental implant assembly according to the at least another example embodiment.
Fig. 5 is a top view of the fixture of a dental implant assembly according to Figs. 3 and 4.
Figs. 6a-6c illustrate at least one example embodiment of how to carry out at least one embodiment of the inventive method.
Fig. 7 illustrates alternative method steps for carrying out at least some example embodiments of the inventive method.
Fig. 8a illustrates a dental implant assembly according to at least one other example embodiment.
Fig. 8b is a cross-sectional view of the fixture/driver interface of the dental implant assembly in Fig. 8a.
Fig. 9 illustrates a dental implant assembly according to at least one further example embodiment.
Fig. 10 illustrates schematically a dental fixture according to at least one example embodiment of the invention, which may be used in a dental implant assembly and/or method according to at least one example embodiment of the invention.

### Detailed description of the drawings

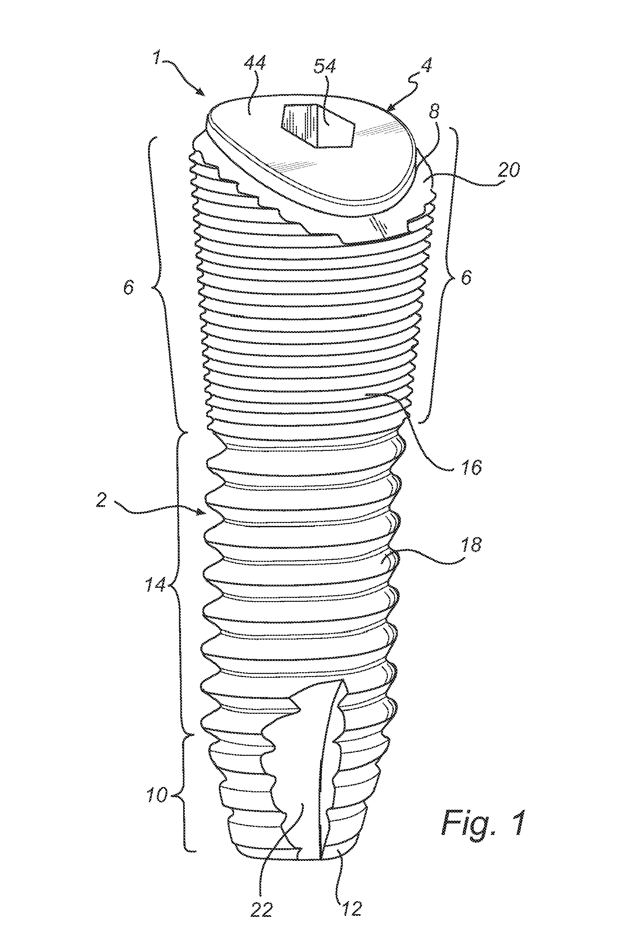

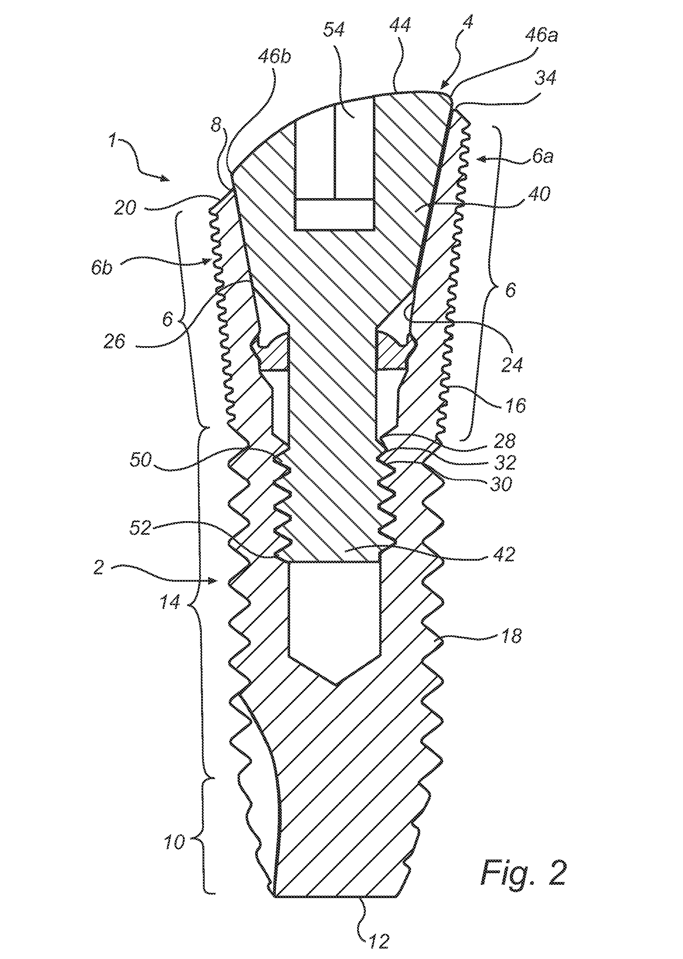

Fig. 1 illustrates a dental implant assembly 1 according to at least one example embodiment of the invention and Fig. 2 is a cross-sectional view of the implant assembly 1 in Fig. 1.

The implant assembly 1 comprises a dental fixture 2 and a dental component 4, herein illustrated as a healing cap or cover screw.

The herein illustrated fixture 2 has a coronal portion 6 extending apically from a coronal end 8 of the fixture, and an apical portion 10 extending coronally from an apical end 12 of the fixture 2. An intermediate portion 14 extends between the coronal portion 6 and the apical portion 10.

The apical portion 10 has a conicity tapering towards the apical end 12 of the fixture 2 to ease insertion of the fixture 2 into a bore-hole in the jawbone. The angle of taper relative to the longitudinal axis of the fixture 2 may, for instance, be about 10°-20°, such as 15°. Although Fig. 1 illustrates a threaded apical portion 10, in an alternative embodiment the apical portion of the fixture may be non-threaded. Whether provided with thread or not, the apical portion 10 may optionally, similarly to the coronal and/or the intermediate portion, further be provided with a blasted, etched or otherwise roughened surface structure.

The fixture 2 has a core from which a surface structure projects, in the illustrated example being in the form of threads.

The coronal portion 6 is herein illustrated as being at least partly provided with microthreads 16, having three thread spirals, although another number is conceivable, such as 1, 2, 4 or more spirals. Although microthreads 16 have been illustrated, according to at least an alternative example embodiment the coronal portion 6 is at least partly provided with macrothreads, similarly to the intermediate portion 14, either as a separate thread spiral or as a continuation of the thread spiral at the intermediate portion 14. According to at least another alternative example embodiment, instead of microthreads 16, the coronal portion 6 may be provided with a plurality of annular ridges, which to the naked eye could give the same visual appearance as microthreads. Other conceivable alternatives are circumferential lines of beads or non-oriented/randomly provided projections such as bulges. Another alternative is to provide the coronal portion with a smooth surface instead of the microthreads.

In the illustrated example embodiment, the macrothread 18 at the intermediate portion 14 has the same lead as the microthreads 16 at the coronal portion 6. However, the pitch of the macrothreads 18 is three times the pitch of the microthreads 16, since the microthreads 16 comprise three thread spirals.

The average axial length of the herein illustrated coronal portion 6 may be about 1-6 mm, such as 3 mm. However, shorter or longer lengths are readily conceivable. The relative average axial length of coronal portion 6 may also be selected from a wide range, such as 5-50% of the total length of the fixture 2, e.g. 10-20%.

The coronal portion 6 comprises a tapering end portion 20, which tapers towards the coronal end 8 of the fixture 2. The tapering end portion 20 is no more than 4% of the total length of the fixture 2. The surface of the tapering end portion 20 may be non-threaded, either smooth or blasted (or otherwise roughened).

The intermediate portion 14 comprising macrothreads 18 is herein illustrated as having one thread spiral, however, the intermediate portion 14 may alternatively have two or more thread spirals. Similarly, although illustrated as having a substantially straight cylindrical shape, the intermediate portion 14 may have a slightly tapering shape towards the apical portion, in which case the angle of taper may e.g. be 3° or less, such as about 1°-2°.

Cutting recesses 22 or grooves extend from the apical end 12 into the intermediate portion 14. The number of cutting recesses 22 may be one or more, such as two, three or four cutting recesses, suitably symmetrically positioned about the circumference of the apical end 12 of the fixture for self-tapping of the fixture 2 when being screwed/rotated into a bore-hole provided in the maxilla or mandible.

A socket or internal bore 24 having an open end is provided in the coronal end of the fixture. The internal bore 24 extends apically into the fixture. The internal bore 24 is for receiving a dental component 4 such as the illustrated cover screw which will be present during an initial healing period. However, it may also receive other dental components such as an abutment, abutment replica and an impression pick-up element.

Although various alternative configurations are conceivable, the internal bore 24 is herein illustrated as having a conical coronal section 26 The internal bore 24 is further provided with an internally threaded apical section 28 comprising a first thread 30 with a first thread start 32.

The fixture 2 has a sloped coronal end 8, wherein a lingual side 6a has a longer extension than a buccal side 6b. When the fixture 2 has been installed in a bore hole in the jawbone, the coronal end 8 should follow the sloped contour of the jawbone. Thus, the fixture 2 has a desired rotational orientation relative to the jawbone.

The lingual side 6a of the sloped coronal end represents a well-defined feature, namely the highest or most coronal area of the fixture 2. Thus, in this embodiment the distinguishable fixture index 34 is the most coronal area of the fixture 2. This fixture index 34 has a known rotational position in relation to the first thread start 32. Thus, when the dentist inserts the dental fixture 2 into the bone and follows the recommendation that the sloped top should be installed such that the side 6a with the longest axial extension of the fixture 2, including said coronal-most area (the fixture index 34), is located lingually while the side 6b with the shortest axial extension of the fixture 2 is located buccally, then said first thread start 32 will be arranged at a defined rotational orientation with respect to the jawbone.

The dental component 4 is made in one piece. It has a body portion 40 and a screw portion 42. The body portion 40 has a sloped top 44 which matches the sloping of the coronal end 8 of the fixture 2. Thus, the body portion 40 has a longer axial extension on the lingual side 46a compared to the buccal side 46b. With respect to an axial plane having the lingual side 46a of the body portion 40 on one side of the plane and the buccal side 46b on the other side of the plane, the dental component 4 is asymmetrical because of the difference in axial extension. Furthermore, because of the sloped top 44 of the body portion 40, the body portion 40 is axially asymmetrical.

The screw portion 42, located at the apical end of the dental component 4, is provided with a second thread 50 having a second thread start 52 for engagement with said first thread 30 in the fixture 2. Thus, it is understood that said first thread start 32 is located at the coronal end of the first thread 30, while said second thread start 52 is located at the apical end of the second thread 50.

Said second thread start 52 has a defined rotational position in relation to the asymmetrical body portion 40. Expressed differently, the sloped top 44 has a known rotational position in relation to said second thread start 52. A simple example would be to have the coronal-most portion of the sloped-top and the second thread start 52 at the same angular position with respect to the axis of the dental component.

The coronal end of the dental component 4 is provided with a socket such as an internal hexagonal recess 54, for receiving an insertion tool, such as an Allen key. The dentist is thus able to rotate the dental component 4 into the fixture 2 by means of the insertion tool. The hexagonal recess 54 is just an example, and the skilled person readily understands that other alternative tool-connecting interfaces may be implemented.

The rotational position of the first thread start 32 in relation to the fixture index 34 and the rotational position of the second thread start 52 in relation to the asymmetrical shape of the dental component 4 have been designed such that when the dental component 4 has been fully install in the fixture 2 the sloped top 8 of the fixture 2 and the sloped top 44 of the dental component 4 will slope in the same direction.

Thus, as long as the dentist orients the fixture 2 correctly with respect to the jawbone, the dental component will when fully installed, without any further consideration, also become correctly oriented with respect to the jawbone.

It should be understood, that an alternative fixture index could be "the most apical area of the coronal end of the fixture", i.e. at the buccal side 6b. Since there is only one such area, this way of identifying a fixture index would also result in a distinguishable fixture index.

The external macrothread 18 of the fixture 2 is also provided with a thread start. During manufacturing, this thread start may also be arranged in a defined rotational orientation with respect to said distinguishable fixture index 34. Because the external screw thread 18 has a known lead and the fixture 2 has a known length, the dentist can be instructed regarding which rotational orientation the fixture 2 should have relative the jawbone when starting to rotate the fixture 2 into the jawbone, in order to arrive with the sloping coronal top 8 substantial flush with the crest of the jawbone. It should be noted that although a defined rotational orientation between the external thread start and said fixture index may be advantageous, it is merely an option. Without such a relationship, the dentist may simply adjust the rotational orientation, with the potential effect that the sloped top may become located slightly above or slightly below the crest of the jawbone.

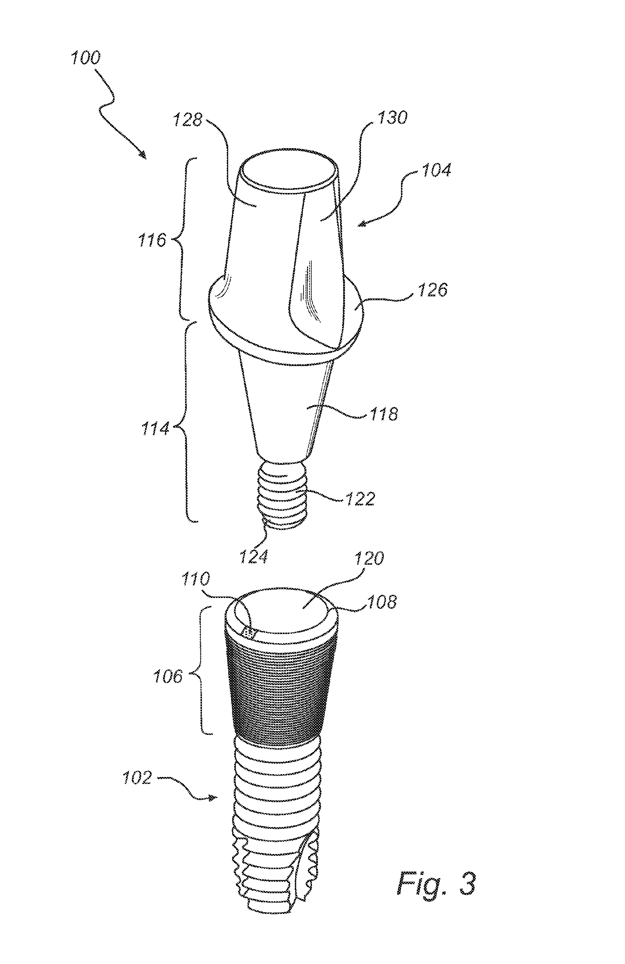

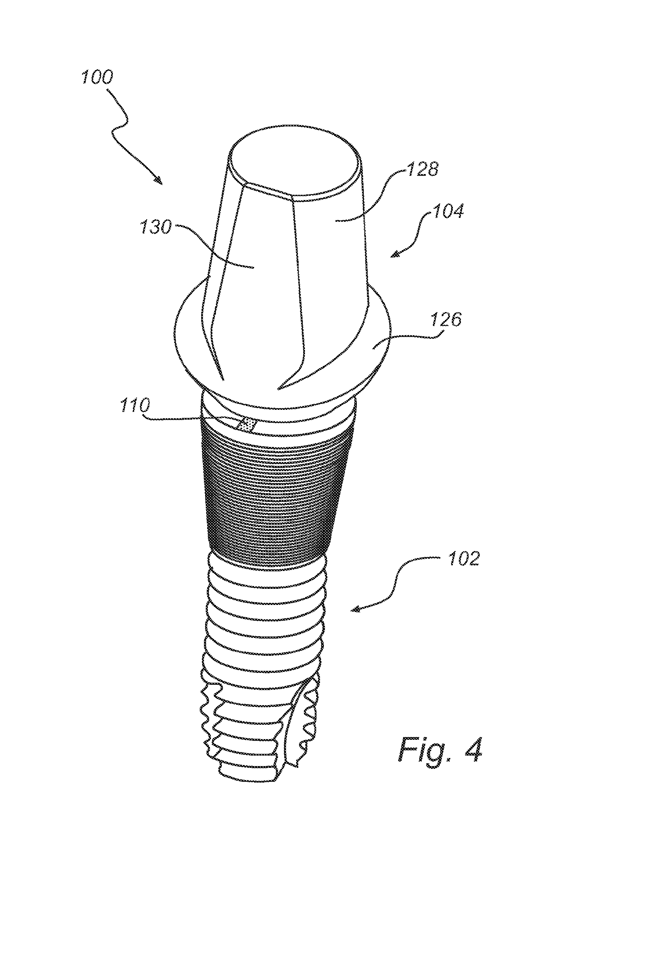

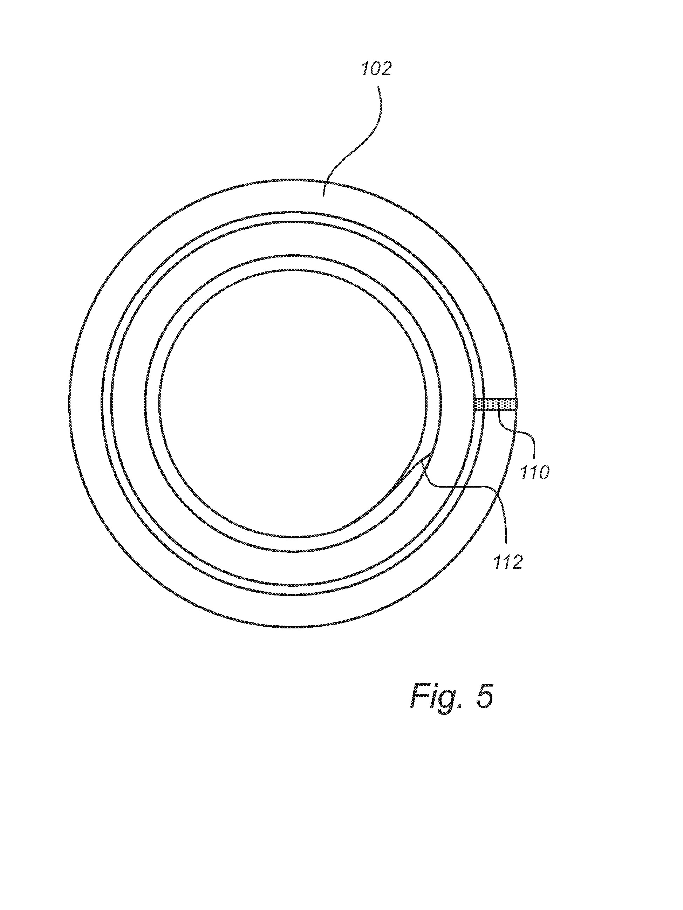

Figs. 3 and 4 illustrate a dental implant assembly 100 according to the at least another example embodiment. The dental implant assembly 100 comprises a dental fixture 102 and a dental component, herein illustrated as an abutment 104. Fig. 5 is a top view of the fixture 102 of a dental implant assembly according to Figs. 3 and 4.

Fig. 3 illustrates the fixture 102 and the abutment 104 before assembling, while Fig. 4 illustrates the fixture 102 and the abutment 104 in a fully/final assembled state.

Unlike the slop-topped fixture 2 in Figs. 1 and 2, the fixture 102 in Figs. 3 and 4 has a flat coronal end 108 (see Fig. 3). In other word there is no difference between a lingual side and buccal side as regards axial extension of the coronal fixture portion 106.

The fixture 102 is provided with a visual marking 110, which may be a colour marking or a surface roughening. The visual marking serves as a distinguishable fixture index 110 which is distinguishable from other markings, if any. Thus, the dentist may be instructed to insert the fixture 102 into the bone so the distinguishable fixture index 110 faces a certain direction, e.g. buccally. The distinguishable fixture index 110 has a defined rotational position relative to the thread start 112 (see top view of Fig. 5) of the internal thread of the fixture 102.

The dental component is illustrated as an abutment 104. The abutment 104 has an apical engagement portion 114 and a coronal prosthesis-receiving portion 116. The engagement portion 114 has an apically tapering conical surface 118 adapted to mate with a corresponding internal surface in the internal bore 120 of the fixture 102. Apically of said tapering conical surface 118, the engagement portion 114 is provided with a thread 122, herein referred to as the second thread, which has a thread start 124, herein referred to as the second thread start.

The prosthesis receiving portion 116 has a sloping shoulder 126, which should suitably be arranged in the oral cavity so that the upper or coronal-most area of the shoulder is arranged lingually while the lower area of the shoulder is arranged buccally. Thus, the sloping shoulder 126 results in an asymmetrical shape of the abutment 104. In this example embodiment, a truncated conical post 128 extending coronally from the shoulder 126 has a flat side 130 which may provide a rotational lock for the prosthesis. This flat side 130 also presents an axial asymmetry of the abutment 104. The asymmetrical shape of the abutment 104 has a known or defined rotational position in relation to the second thread start 124. For instance, following the circumference around the axis of the abutment, the flat side 130 may be known to be displaced approximately 90° from the location of the second thread start 124. This is of course just an example, and other angular relationship are equally conceivable.

When the abutment 104 is to be connected to the fixture 102, the second thread start 124 will mesh with the first thread start 112 (Fig. 5), and the abutment 104 will be rotated into the fixture 102. The rotational positions of the first thread start 112 and second thread start 124 in relation to the fixture index 110 and the asymmetrical shape of the abutment (e.g. the sloping of the shoulder 126 or the flat side 130), respectively, have been configured such that when the conical surface 118 of the engagement portion 114 of the abutment 104 lands against the corresponding conical surface in the fixture 102, the rotational orientation of the asymmetrical shape will be in a pre-defined relation to the fixture index 110. In the illustrated example embodiment, the predefined rotational orientation of the asymmetrical shape is such that the flat side 130 and the downward sloping of the shoulder 126 on the prosthesis-receiving portion 116 are aligned with the angular position of the distinguishable fixture index 110 (see Fig. 4). Thus, in order to get the suggested orientation of the shoulder portion 126 (sloping from lingual to buccal), the dentist simply inserts the fixture 102 into the jawbone of the patient, making sure that the fixture index 110 is facing buccally, and then the abutment 104 will by design arrive in the suggested rotational orientation when fully connected to the fixture 102.

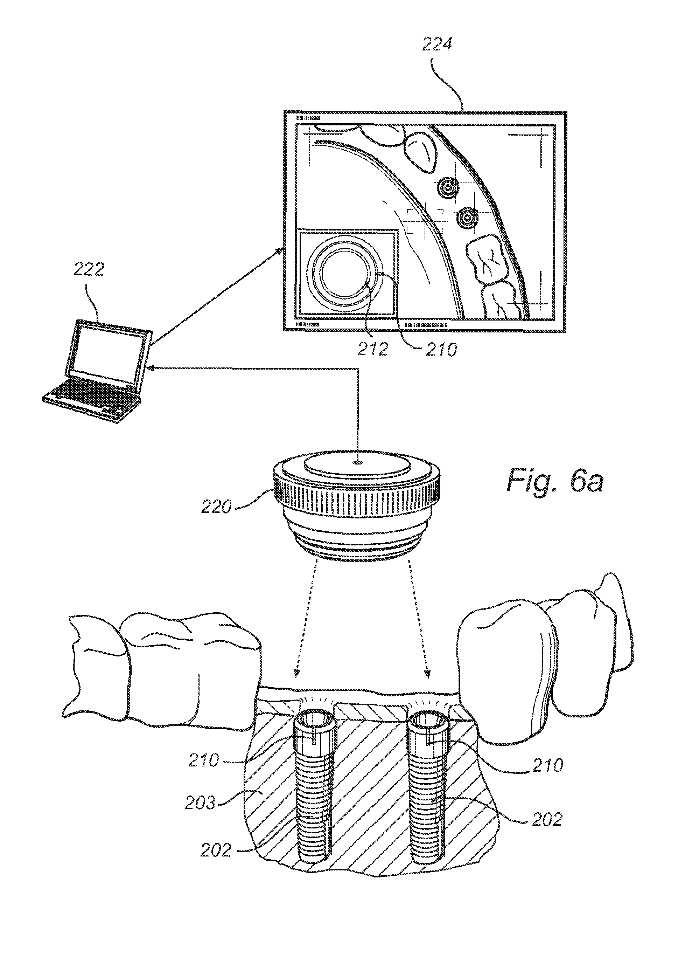

Figs. 6a-6c illustrate at least one example embodiment of how to carry out at least one embodiment of the inventive method.

In Fig. 6a, two dental fixtures 202 are shown as implanted flush with bone tissue 203 at an implantation site (preparation site) between adjacent teeth. Each fixture 200 is provided with a distinguishable fixture index 210, herein illustrated as a marked line extending on the outer and coronal end of the fixture. However, it should be understood that said fixture index 210 could be designed differently, as exemplified elsewhere in this application. It should also be understood that although submerged fixtures 200 having sockets are illustrated, other alternative fixtures are possible as well, for instance, transgingival fixtures having a head portion extending above the bone tissue.

In Fig. 6a, a digital impression is made by scanning the site, e.g. by means of a 3D camera 220, thus the rotational orientation of said fixture index 210 is observed in relation to the surrounding teeth and jawbone 203. The digital impression or optical image can be imported and/or stored to a computer 222 as a three dimensional image 224, from which the inclination of the fixtures 202 and the rotational position of the fixture index 210 are identifiable. The fixture index 210 has a defined rotational position in relation to the thread start 212 of the internal fixture thread, herein referred to as the first thread start 212.

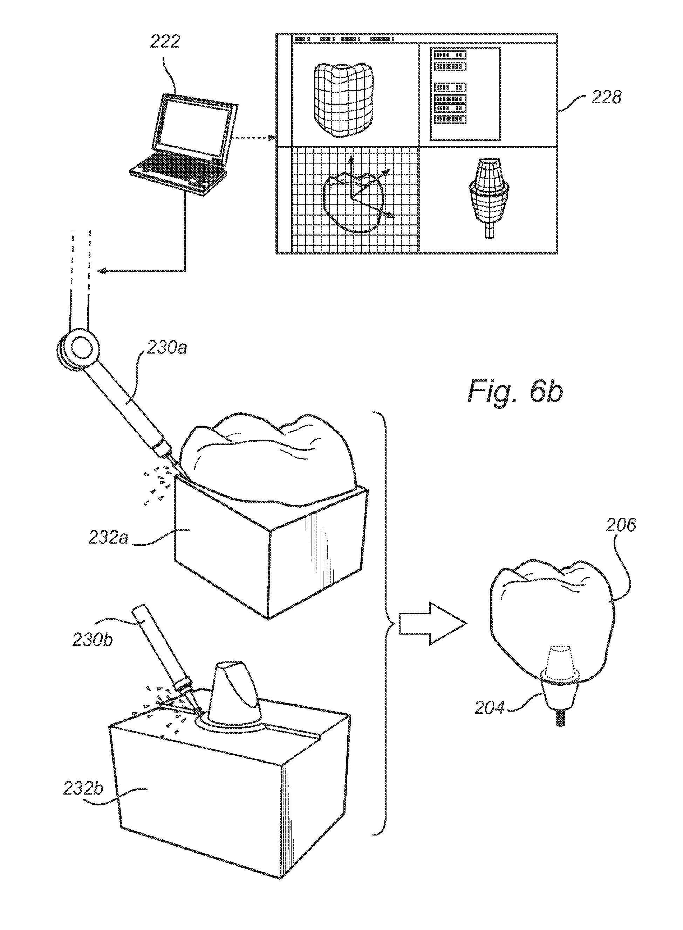

As illustrated in Fig. 6b, by means of computer aided design a desired shape of the abutment 204 and prosthesis 206 is formed. The patient-specific abutment 204 will have an asymmetrical shape which is intended to be oriented in a certain direction relative to the surrounding tissue when in place in the oral cavity. To achieve this, the rotational position of the asymmetrical shape of the abutment is adapted to the thread start (herein the second thread start) of the abutment, such that when the abutment 204 is fully installed the asymmetrical shape will automatically be oriented as intended. Thus, because said fixture index 210 provides information about the rotational orientation of the first thread start 212, and the second thread start at the abutment 204 will engage with the first thread start 212 so as to allow the abutment 204 to be rotated into the fixture 202 a known distance (known number of full and partial rotations), the asymmetrical shape can be designed accordingly.

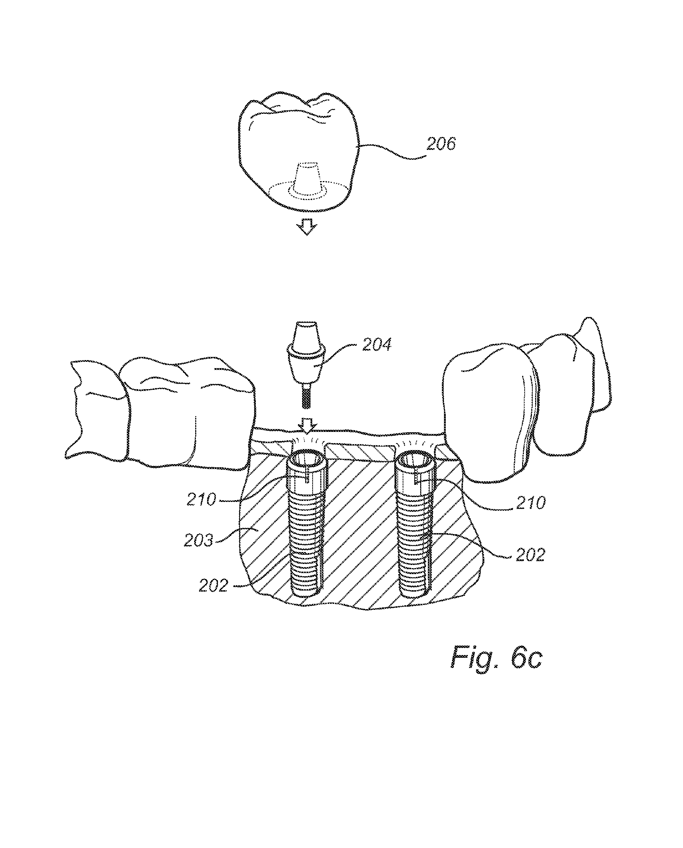

When the abutment 204 and prosthesis 206 have been designed 228, control data is provided to manufacturing equipment to form the components. Here, the forming of the respective component is illustrated as a process 230a, 230b of cutting away material from a blank 232a, 232b resulting in a prosthetic tooth 206 and an abutment 204. However, other manufacturing methods are conceivable as well, one example being laser sintering. Finally, as illustrated in Fig. 6c, the formed abutment 204 is by means of its external thread connected to a fixture 202 in the jawbone 203. The prosthesis 206 is connected to the abutment 204. Because the designed patient-specific abutment 204 has a known end-position in the fixture 202, which is designed based upon a digital impression which provides a three dimensional image mimicking the fixture in the jawbone, the risk of the dentist incorrectly orienting the abutment with respect to the fixture is considerably reduced.

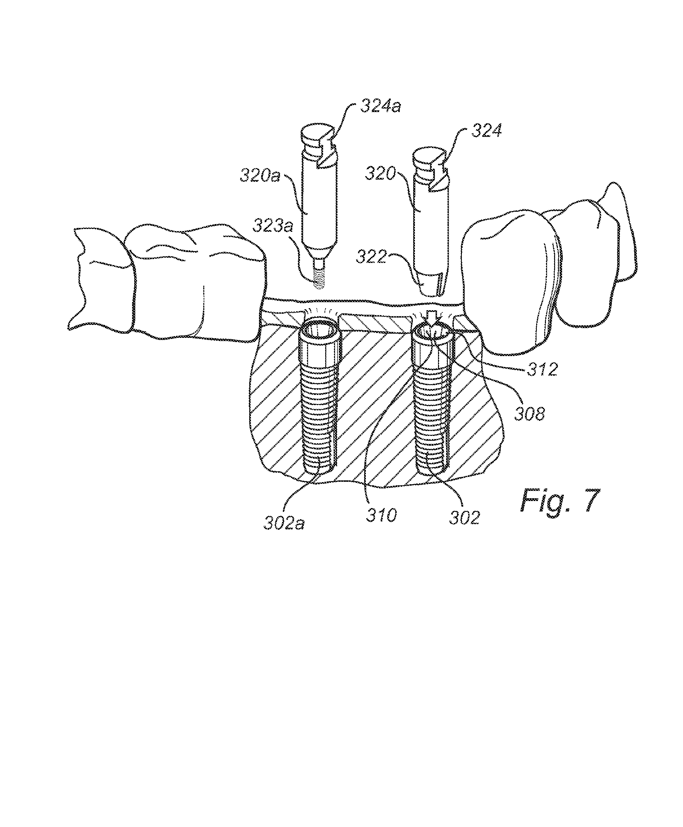

It should be understood that although Figs. 6a-6c have illustrated acquiring a digital impression, an alternative would be to make a traditional physical impression in which case a first index should be such that it can be transferred to a model of the jawbone with fixture replicas. An example of such a solution is shown in Fig. 7.

Fig. 7 illustrates alternative method steps for carrying out at least some example embodiments of the inventive method.

With reference to the fixture 302 on the right hand side in Fig. 7, in this example embodiment, the internal bore of the fixture 302 is provided with an engaging portion 308. The engaging portion 308 has a circumferential wall 310 which is interrupted by a radial recess 312. The position of the recess 312 is defined/known with respect to the thread start (not shown) inside the fixture 302. Thus, the recess 312 could be regarded as a distinguishable fixture index 312. However, in order to make a traditional impression of the implantation site, it may be advantageous to use a connectable impression or observation component 320. The observation component 320 has a complementary engagement portion 322 which can only mate with the engaging portion 308 of the fixture 302 in one pre-defined rotational orientation. The observation component 320 may have an asymmetrical shape, such as the herein illustrated coronal end portion, thereby presenting an observation component index 324. The observation component index 324 has a known rotational orientation relative to the engagement portion 322. Thus, because, the observation component 320 can only engage the fixture 302 in one rotational orientation, the rotational orientation of the observation component index 324 relative to the first thread start of the fixture 302 is also known. Thus, when making a traditional impression, the impression mould will have the corresponding features and a model can be made which mimics the real implantation site. A dental component, for instance an abutment, can then be designed based on the model, making it possible to arrive at an end result corresponding to that in Fig. 6c. Alternatively, rather than making a traditional physical impression, the observation component 320 in Fig. 7 may be used as a scan abutment, allowing a digital impression to be made which very clearly indicates the rotational orientation of the observation component index 324 and therefore inherently the rotational orientation of the thread start. This information can then be used for making an abutment and prosthesis, for instance, as exemplified in Fig. 6b.

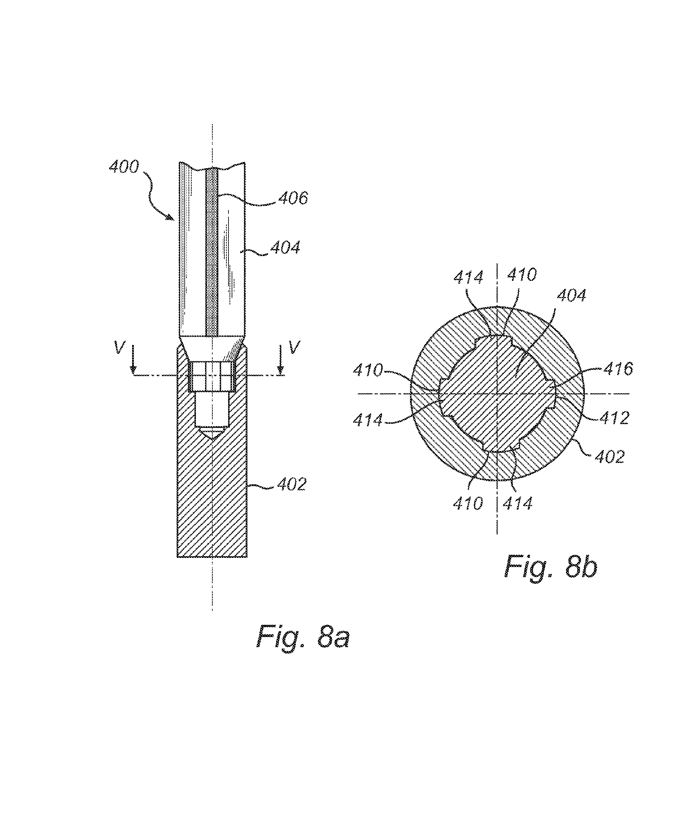

Although the recess 312 in the engaging portion 308 of the fixture 302 in Fig. 7 may be regarded as a fixture index, it may (e.g. depending on the size of the recess) be difficult for the dentist to accurately see the fixture index when installing the fixture into the jawbone. Therefore, following the corresponding principle as for the observation component 320, an installation device having a pre-defined single mating position with the fixture and a distinguishable installation device index may be used. In such case, the dentist can observe the rotational orientation of the installation device index to determine when the fixture is in a correct rotational orientation relative to the bone tissue. An example of such an installation device is shown in Fig. 8a.

With reference to the fixture 302a on the left hand side in Fig. 7, the fixture 302a does not have a recess in the engaging portion. However, an observation component 320a having a distinguishable observation component index 324a and a thread 323a may be screwed into the internal threaded bore of the fixture 302a. Because, the rotational position of the thread start of the thread 323a of the observation component 320a is known in relation to the observation component index 324a, the rotational orientation of the first thread start in the fixture 302a (in relation to the surrounding anatomy) may be deduced when the observation component 320a has been fully installed in the fixture 302a. However, the dental technician does not necessarily have to know the rotational orientation of the first thread start, but could simply provide a dental component having a threaded portion corresponding to the threaded portion 323a of the observation component 320a. Because the rotational orientation of the thread start of the observation component 320a in the fully installed state is deducible, by having a corresponding threaded portion on the dental component the dental technician will know that the second thread start of the dental component will in the fully installed state of the dental component, have the same rotational orientation. Based on this, the dental technician can then design an appropriate asymmetrical shape of the patient-specific dental component so that the rotational orientation of said asymmetrical shape will be correctly oriented when the dental component has been fully installed.

Fig. 8a illustrates a dental implant assembly 400 according to at least one other example embodiment. An installation device 404, e.g. a driver, such as illustrated in Fig. 8a, may be provided with a distinctive visual marking 406 and be received in only one rotational orientation relative to the fixture 402. The marking will function as an installation device index 406 which will thus indicate to the user the direction of the first thread start (not shown) in the internal bore of the fixture. The final rotational position of the fixture 402 may be easily adjusted when looking at the distinctive marking 406.

There are, of course, many ways to design an interface between the fixture 402 and the driver 404, for allowing only one mating position. One such example is shown in Fig. 8b, which is a cross-sectional view (along line v-v) of the fixture/driver interface of the dental implant assembly 400 in Fig. 8a. The engaging portion of the fixture 402 is provided with three large recesses 410 and one smaller recess 412. The driver 404 has three large protrusions 414 which can only fit into the large recesses 410, and not into the small recess 412. The driver 404 also has a small protrusion 416 which fits into the small recess 412. Thus, the driver protrusions and recesses will provide four driving surface.

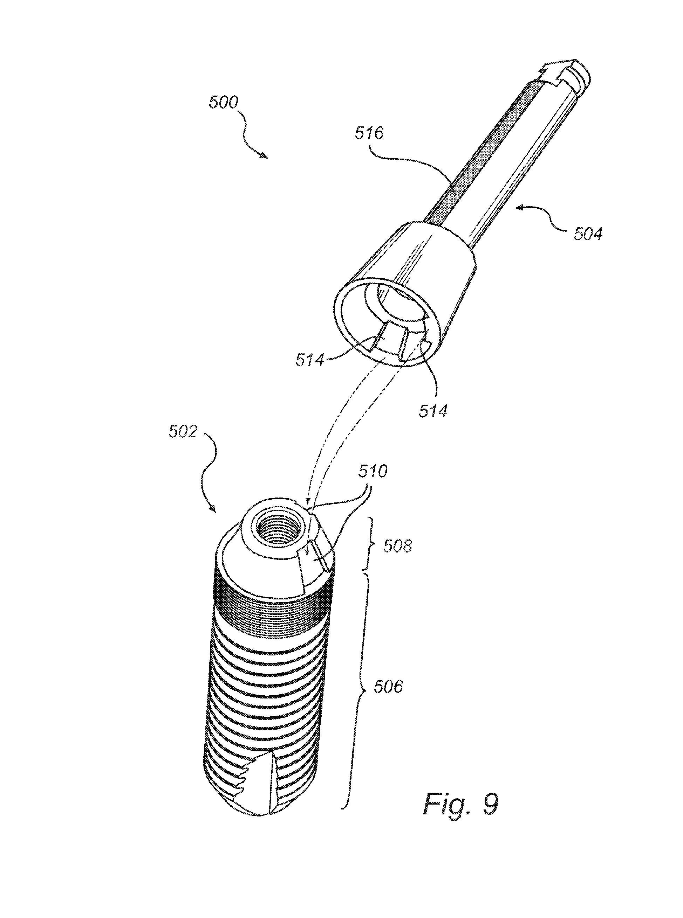

An alternative interface is shown in Fig. 9 which illustrates a dental implant assembly 500 according to at least one further example embodiment. The dental implant assembly 500 comprises a fixture 502 which can receive a dental component or an installation device 504 in only one rotational orientation. In this example a transgingival fixture 502 is illustrated. Thus, similarly to the previously illustrated fixtures, the present fixture 502 has a bone apposition portion 506 adapted to be submerged into the bone tissue. Additionally, the present fixture has a summit portion 508 adapted to be located outside the bone. The installation device 504 is herein illustrated in the form of a driver. The installation device 504 has only two (herein illustrated as equally-dimensioned) radial protrusions 514. However, contrary to the embodiment shown in Figs. 8a and 8b, in which the radial protrusions are located on an outer perimeter of the installation device, in Fig. 9, the radial protrusions 514 are located on an inner perimeter of the installation device 504, the inner perimeter defining an interior space of the installation device 504 which is adapted to fit over the summit portion 508 of the fixture 502. The summit portion 508 of the fixture 502 has two recesses 510 configured and dimensioned to mate with the radial protrusions 514.

As may be seen in Fig. 9, the installation device 504, herein exemplified as a driver has a distinctive marking 516, i.e. an installation device index 516.

Because
a) the radial recesses 510 on the fixture 502 are positioned in a known or pre-defined rotational position in relation to the thread start of the internal fixture thread,
b) the installation device 504 can only mate correctly with the fixture 502 in one rotational orientation relative to the fixture 502, and
c) the installation device index 516 has a know rotational position in relation to the radial protrusions 514 which can only mate in one rotational orientation in relation to the radial recesses 510,
   the installation device index 516 will, when the installation device 504 is correctly connected to the fixture 502, have a known or predefined rotational orientation relative to said thread start.

Thus, if an axially asymmetrical dental component to be subsequently attached to the fixture 502 should have a certain orientation with respect to the surrounding jawbone and teeth etc, and that dental component has (as previously exemplified) a second thread start which is arranged such that the asymmetrical shape of the dental component will be in a known rotational orientation with respect to e.g. the summit portion 508 of the fixture 502, then the dentist can control and anticipate said certain orientation by ending the insertion of the fixture 502 when the installation device index 516 is in a correct rotational orientation with respect to the surroundings.

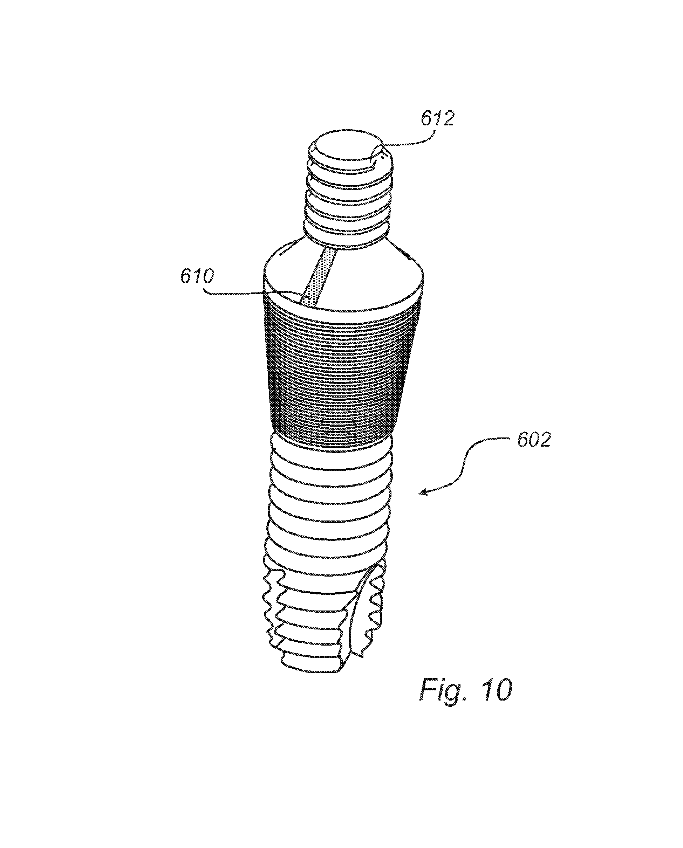

Fig. 10 illustrates schematically a dental fixture 602 according to at least one example embodiment of the invention, which may be used in a dental implant assembly and/or method according to at least one example embodiment of the invention. The fixtures in the previous drawings have illustrated an internal bore provided with a first thread. Fig. 10, however, illustrates a transgingival dental fixture 602 having a summit portion, and on the exterior surface of the summit portion, the fixture 602 comprises a first thread having a first thread start 612. The fixture 602 is also provided with a distinguishable fixture index 610, wherein the rotational position of the fixture index 610 in relation to said first thread start 612 is known. A dental component having a second thread with a second thread start on an interior surface of the dental component may thus be engaged to the fixture 602. As in the previous example embodiments, the rotational position of an asymmetrical shape of such a dental component may be adapted to the second thread start so that, when the dental component is fully installed, the asymmetrical shape of the dental component will have a desired rotational orientation in relation to the surrounding anatomy.

## Claims

1. A dental implant assembly, comprising
a fixture for insertion into a jawbone which is provided with a first thread having a first thread start, and
an axially asymmetrical dental component, such as an abutment, an abutment replica, a driver, a healing cap or an impression pick-up element, which is provided at its apical end with a second thread with a second thread start having a rotational position which is known in relation to the asymmetrical shape of the dental component for engagement with the first thread,
wherein the fixture is provided with a distinguishable fixture index which has a known rotational position in relation to the first thread start,
wherein, when the fixture has been inserted into the jawbone and the dental component has been fully installed therein, the rotational orientation of the fixture index in relation to the surrounding anatomy determines the rotational orientation of the asymmetrical shape of the dental component in relation to the surrounding anatomy.

2. A dental implant assembly according to claim 1, wherein said fixture index is provided on an exterior of said fixture.

3. A dental implant assembly according to claim 2, in which said fixture index is located at a coronal end of the fixture.

4. A dental implant assembly according to claim 3, in which said fixture index is chosen from the group consisting of: an axially asymmetrical coronal fixture profile, a recess, a protrusion, an indentation, a local surface modification and a colour marking.

5. A dental implant assembly according to claim 1, wherein said fixture index is comprised in an internal bore provided at a coronal end of the fixture.

6. A dental implant assembly according to claim 5, wherein said fixture index is an asymmetrical irregularity in a surface of the internal bore.

7. A dental implant assembly according to any one of the preceeding claims, comprising at least two dental components, wherein the rotational position of the second thread start is different between the at least two dental components, in relation to their respective asymmetrical shapes, thereby enabling said asymmetrical shapes to be positioned in a respective rotational orientation relative to the fixture when the dental components are fully installed therein.

8. A dental implant assembly according to any one of the preceeding claims, in which said dental component is a one-piece component.

9. A dental implant assembly according to any one of the preceeding claims, in which said fixture is provided with external threads for engagement with a bore in said jawbone, and wherein said external threads have a thread start having a rotational position which is known in relation to the fixture index.

10. A dental implant assembly, comprising
a fixture for insertion into a jawbone, which is provided with an engaging portion having a first thread with a first thread start having a rotational position which is known in relation to the engaging portion, and
an installation device, such as a driver, which is provided at an apical end with a first engagement portion which is adapted to mesh in a pre-defined rotational orientation with said engaging portion of the fixture during installation thereof, the installation device further comprising a distinguishable installation device index having a known rotational position to the first engagement portion.

11. A dental implant assembly according to claim 10, wherein the first engagement portion has a third thread having a third thread start having a rotational position for engagement with the first thread.

12. An axially asymmetrical dental component, such as an abutment, an abutment replica, a healing cap or an impression pick-up element, to be installed in a fixture which is installed in or is to be installed in a jawbone of a patient, said fixture being provided with a first thread having a first thread start, wherein the first thread start has a known rotational position in relation to a distinguishable fixture index,
the dental component being provided at an apical end with a second thread with a second thread start having a rotational position which is known in relation to the asymmetrical shape of the dental component, for engagement with the first thread, the position of the second thread start being adapted to the position of the first thread start such that prior to installation of the dental component in the fixture the rotational orientation between the asymmetrical shape of the dental component and the fixture index after final installation is known.

13. A dental component according to claim 12, in which said dental component is a one-piece component.

14. A method for manufacturing an axially asymmetrical dental component, such as an abutment, an abutment replica, a healing cap or an impression pick-up element, which is to be installed in a fixture which is installed in or is to be installed in a jawbone of a patient, said fixture being provided with a first thread with a first thread start, the method comprising the steps of:
- providing the dental component at an apical end with a second thread with a second thread start having a rotational position which is known in relation to the asymmetrical shape of the dental component, for engagement with the first thread, and
- adapting the second thread start to the position of the first thread start such that a desired rotational orientation between the asymmetrical shape of the dental component and the fixture after final installation is achieved.

15. A method according to claim 14, comprising
- providing an observation component comprising a distinguishable observation component index and a thread with a thread start, wherein the rotational position of the thread start of the observation component is known in relation to the distinguishable observation component index, wherein the thread of the observation component is adapted to engage with said first thread of the fixture for installing the observation component into or onto the fixture,
- observing, when the observation component has been fully installed in/on the fixture installed in the jawbone, the rotational orientation of the observation component index in relation to the surrounding anatomy, wherein said step of adapting the second thread start to the position of the first thread start is based on the observed rotational orientation of the observation component index in relation to the surrounding anatomy.

16. A method according to claim 14, wherein the fixture is provided with a distinguishable fixture index having a known relationship to the first thread start, the step of manufacturing the dental component being preceded by the steps of:
- observing a rotational orientation of said fixture based on said fixture index,
- determining a desired rotational orientation of said asymmetrical shape of the dental component in relation to the fixture when fully installed therein,
- selecting the rotational position of the second thread start of the dental component corresponding to the desired rotational orientation of said dental component in relation to the fixture.

17. A method for manufacturing an axially asymmetrical dental component, such as an abutment, an abutment replica, a healing cap or an impression pick-up element, which is to be installed in a fixture which is installed in a jawbone of a patient, said fixture being provided with an engaging portion having a first thread with a first thread start, the method comprising the steps of:
- observing the rotational position of said first thread start by using an observation component which is installed in said fixture when said fixture has been installed in the jawbone of the patient, said observation component being provided at an apical end with an engagement portion which is adapted to mesh in a pre-defined rotational orientation with said engaging portion of the fixture, the observation component further comprising a distinguishable observation component index having a known rotational position in relation to the engagement portion,
- determining a desired rotational orientation of said asymmetrical shape of the dental component in relation to the fixture when fully installed therein,
- selecting the rotational position of the second thread start of the dental component corresponding to the desired rotational orientation of said asymmetrical shape of the dental component in relation to the fixture,
- providing the dental component at an apical end with a second thread with a second thread start having the selected rotational position in relation to the dental component.

18. A dental fixture for insertion into a jawbone, which is provided with a first thread having a first thread start having a rotational position, wherein the fixture is provided with a distinguishable fixture index which has a known rotational position in relation to the first thread start.

19. A dental fixture according to claim 18, in which said fixture index is chosen from the group consisting of: an asymmetrical coronal fixture profile, a recess, a protrusion, an indentation, a surface modification and a colour marking.

20. A dental implant assembly according to any one of claims 10 or 11, or a method of manufacturing according to any one of claims 14 to 17 or a dental fixture according to any one of claims 18 to 19, wherein the fixture comprises the features of the fixture in any one of claims 1 to 9.
